# EUROPÄISCHE PATENTANMELDUNG

(11) **EP 2 378 119 A1**
(43) Veröffentlichungstag der Anmeldung: **19.10.2011**
(21) Anmeldenummer: 10160010.4
(22) Anmeldetag: 15.04.2010
(51) Int. Cl.: F04B 19/00

(54) **Plungerpumpe mit manueller Eingriffsmöglichkeit für Volumen unter einem Mikroliter**

(71) Anmelder: MMI AG, 8152 Glattbrugg (CH)
(72) Erfinder: Tschanz, Peter, 8500 Gerlikon (CH)
(74) Vertreter: Siegert, Georg

(57) **Zusammenfassung**

Eine automatisierte hochpräzise Plungerpumpe für Volumen unter einem Mikroliter, die bei der Mikroskop-gestützten Mikromanipulation von biologischem Material eingesetzt wird, umfasst ein Gehäuse (1) und einen Pumpkopf (8), der einen Arbeitsraum (9) mit einer gemeinsamen oder separaten Ein- und Auslassöffnung (10) definiert, wobei das Volumen des Arbeitsraums (9) durch einen in ihn einbringbaren axial beweglichen Plunger (7) veränderbar ist. Der Plunger (7) ist durch eine Gewindespindel (5) oder eine auf der Gewindespindel (5) in Gewindeeingriff befindliche Spindelmutter (6) axial beweglich ist und die Gewindespindel (5) und/oder die Spindelmutter (6) ist rotatorisch antreibbar und die jeweils andere ist rotatorisch arretierbar ausgelegt.

## Beschreibung

Die vorliegende Erfindung betrifft eine hochpräzise Plungerpumpe mit manueller Eingriffsmöglichkeit für Volumen unter einem Mikroliter gemäß dem Oberbegriff des Anspruchs 1.

### Stand der Technik

Die molekulare Analyse reiner angereicherter Zellkulturen oder sogar einzelner Zellen ist eine wichtige Voraussetzung der medizinischen Genomik und Proteomik, und könnte in Zukunft für eine medizinische Profilierung von Patienten von wesentlicher Bedeutung werden. Bisher war dieses Ziel aufgrund maschineller sowie verfahrenstechnischer Beschränkungen bei der Zellmanipulation (z.B. der Zellsammlung oder der Mikroinjektion) nur schwer und mit nicht unerheblichem Zeitaufwand zu erreichen. Insbesondere die zur genotypischen und phaenotypischen Charakterisierung notwendige Isolierung einzelner sowie seltener Zellen aus winzigen Proben stellte sich sehr schwierig dar, da einzelne Zellen vom Forschungspersonal unter einem Mikroskop erfasst und die erfasste Zelle dann manuell mithilfe von Kapillaren manipuliert werden musste. Hierzu ist es notwendig, in den Kapillaren ein genau definiertes Fluidvolumen zu bewegen, beispielsweise um einzelne Zellen oder Zellinhalte über die Kapillare zu entnehmen oder in eine Zelle Bestandteile anderer Zellen (zum Beispiel Zellkerne) zu injizieren, ohne dabei die Zelle durch zu viel abgesaugtes oder injizierte Material zu beschädigen.

Eine der oben genannten Beschränkungen betrifft insbesondere die zur Volumenabmessung und Bereitstellung des Unter-/Überdrucks eingesetzten Pumpen, die in der Lage sein müssen, mit hoher Präzision Fluid-Volumina der Größenordnung von weniger als einem Mikroliter bis hinab zu einem Nanoliter anzusaugen bzw. auszustoßen. Da aufgrund der kleinen Pumpvolumina und der somit kleinen Pumpenabmessungen die bei Kolbenpumpen nötige Abdichtung des Pumpenkolbens gegenüber dem Pumpenzylinder nur schlecht möglich ist, werden in der Manipulation von Zellmaterial sogenannte Plungerpumpen eingesetzt, bei denen der Plunger lediglich an der Stelle, an der er in den Zylinder eindringt, abgedichtet werden muss.

Um die oben beschriebene Präzision der Volumina bei gleichzeitiger Koordination mit der Zellerfassung unter dem Mikroskop beizubehalten, wird die Erfassung sowie Manipulation der Zellen zum Teil weiterhin manuell betrieben, beispielsweise mit den CellTram-Mikroinjektoren der Firma Eppendorf oder den XenoWorks^{™}-Mikroinjektoren der Firma Butter Instrument, bei denen der Plunger durch Drehen eines Handrads betätigt wird. Ein derartiges manuelles Vorgehen bei der Zellmanipulation ist natürlich äußerst zeitintensiv und eignet sich somit immer weniger für die stetig zunehmende Anzahl an zu verarbeitenden Proben, nicht zuletzt aufgrund der nötigen Personalkosten.

Andererseits ist bekannt, die eben genannten manuellen Pumpen über einen am Handrad angekoppelten Antriebsmotor zu betreiben. Ein weiterer Versuch der Automation des Manipulationssystems wurde mit den Mikroinjektionspipetten Nanoject II der Firma Drummond Science unternommen, bei denen der Plunger direkt an einen mikroprozessorgesteuerten Elektromotor gekoppelt ist. Bei den genannten motorisierten Pumpen besteht allerdings das Problem, dass ihre Antriebe sehr langsam sind und somit Serviceeingriffe, wie beispielsweise das bei jedem Kapillarenwechsel notwendige "Backfilling" mit Fluid, sehr zeitraubend oder sogar unmöglich sind. Auch ist es nicht möglich, die motorisierten Pumpen hierzu auf einfache Weise von Hand zu betreiben, da im Falle der motorisierten Handpumpe der Motor zuerst aufwändig entkoppelt werden muss, während bei der automatisierten Mikroinjektionspipette eine manuelle Betätigung überhaupt nicht vorgesehen ist.

### Darstellung der Erfindung

Es ist somit eine Aufgabe der vorliegenden Erfindung, eine automatisierte Plungerpumpe für Volumen unter einem Mikroliter bereitzustellen, die sowohl automatisch als auch manuell betrieben werden kann, manuelle Eingriffsmöglichkeiten zur Befüllung bzw. Wartung ermöglicht und bei hoher Volumenpräzision mechanisch unempfindlich ist. Diese Aufgabe wird mithilfe einer automatisierten hochpräzisen Plungerpumpe gelöst, die die in Anspruch 1 definierten Merkmale umfasst. Weitere vorteilhafte Ausführungsformen ergeben sich aus den Unteransprüchen.

Die erfindungsgemäße automatisierte hochpräzise Plungerpumpe umfasst ein Gehäuse und einen Pumpkopf, der einen Arbeitsraum mit einer gemeinsamen oder separaten Ein- und Auslassöffnung definiert, wobei das Volumen des Arbeitsraums durch einen in ihn einbringbaren axial beweglichen Plunger veränderbar ist und der Plunger durch eine Gewindespindel oder eine auf der Gewindespindel in Gewindeeingriff befindliche Spindelmutter axial beweglich ist, und die Gewindespindel und/oder die Spindelmutter rotatorisch antreibbar bzw. rotatorisch arretierbar ausgelegt sind.

Durch diesen Aufbau besitzt die erfindungsgemäße Plungerpumpe zwei mechanisch voneinander abkoppelbare Antriebsstränge, da die axiale Bewegung des Plungers entweder durch Antrieb der Gewindespindel oder durch Antrieb der Spindelmutter unabhängig vom jeweils anderen Antriebsstrang bewirkt werden kann. Um den Plunger beispielsweise zum Nachfüllen der Pumpe zu bewegen, ist es somit nicht mehr notwendig, wie im Stand der Technik einen Antriebsstrang aufwändig zu entkoppeln, und es kann aufgrund der direkten Ankopplung des Antriebs an der Gewindemutter eine ebenso präzise wie zu Service- oder Wartungszwecken einfach bedienbare Pumpe bereitgestellt werden. Derartige Service- und Wartungseingriffe sind hierbei nicht nur auf das Nachfüllen von Pumpmedium beschränkt, sondern umfassen unter anderem auch das Spülen oder Reinigen des Arbeitsraumes bzw. der daran anschließbaren Kapillare/Pipette. Es handelt sich bei diesen Eingriffe also um relativ häufig durchzuführende Tätigkeiten, die nun dank der schnelleren und einfacheren Bedienung der Pumpe wesentlich weniger Zeit in Anspruch nehmen.

Insgesamt stellt die Erfindung also eine Plungerpumpe bereit, die mit geringem mechanischen Aufwand eine schnelle, effiziente und somit Kosten senkende Mikromanipulation von biologischem Material ermöglicht, die sich durch Zuverlässigkeit und Sicherheit der mechanischen Teile auszeichnet und die eine herausragende Bedienerfreundlichkeit besitzt, da der Anwender die Möglichkeit hat, in den Prozess der Zellsammlung einzugreifen.

Bevorzugt umfasst dabei die Plungerpumpe als Antrieb einen Motor, der mit der Spindelmutter und/oder der Gewindespindel in Eingriff steht und über sie mit der rotatorisch arretierten Gewindespindel bzw. der rotatorisch arretierten Spindelmutter zum Antrieb des Plungers in Wirkverbindung steht. Wird beispielsweise die Spindelmutter, die drehbar im Gehäuse eingebaut ist, vom Motor angetrieben, so wird die rotatorisch arretierte Gewindespindel, die mit der Spindelmutter in Gewindeeingriff steht, in axialer Richtung bewegt, wodurch auch der mit der Gewindespindel verbundene Plunger bewegt wird und das Volumen des Arbeitsraums der Pumpe verändert.

In einer bevorzugten Ausgestaltung sind die Gewindespindel und/oder die Spindelmutter mit einem vorgeschalteten Getriebe versehen. Dadurch ist es möglich, die axiale Verschiebung des Plungers optimal an die bevorzugte Drehzahl des Motors anzupassen und in Funktion davon zu ändern. Andererseits kann ein derartiges Getriebe (zum Beispiel ein Planetengetriebe) auf einfache Weise einen manuellen Eingriff ermöglichen, das heißt Eingriffspunkte für eine manuelle Drehung des Getriebes bieten, die dann in eine Bewegung des Plungers umgesetzt wird. Als weitere Option könnte auch die im Handantrieb betätigte Gewindespindel mit einer entsprechenden Übersetzung motorisiert werden.

In einer besonders bevorzugten Ausführungsform der Plungerpumpe ist die arretierbare Gewindespindel, die arretierbare Spindelmutter oder der Motor mit einer Kupplung versehen, welche vorteilhafterweise eine Rutschkupplung ist. Diese Kupplung stellt somit ein Sicherheitselement dar und verhindert mechanische Beschädigungen am empfindlichen Antriebsstrang und Motor der Pumpe, sollte es zu übermäßigen Belastungen in der Pumpe kommen, beispielsweise durch Eindringen eines Fremdkörpers in den Arbeitsraum oder durch Fehlbedienung seitens des Benutzers. Die Kupplung kann dabei manuell oder aber auch automatisiert sein.

Der Plunger ist vorzugsweise mit der Gewindespindel mitdrehend verbunden, was eine einfach und kostengünstig zu realisierende Verbindung darstellt. Dies kann zum Beispiel durch einen (lösbaren) Reibschluß zwischen Plunger und Gewindespindel erreicht werden. Insbesondere kann dabei der Plunger auch drehstarr mit der Gewindespindel verbunden sein, was seinen Vorteil darin hat, dass der Plunger sich nicht in der notwendigen Dichtung dreht und somit ein Verschleiß der Dichtung verringert wird.

In einer weiteren vorteilhaften Ausführungsform der Plungerpumpe ist der Pumpkopf mit in den Arbeitsraum führenden, bevorzugt richtungsorientierten Ventilen versehen. Dies erleichtert zusätzlich das Nachfüllen vom Pumpmedium, und die Richtungsorientierung, beispielsweise in Form eines Rückschlagventils, verhindert dabei einen unerwünschten Austritt des Pumpmediums.

Schließlich ist es vorteilhaft, im Pumpenkopf Endschalter vorzusehen, die die Endlagen des Plungers im Arbeitsraum angeben. Diese Endschalter können mit einer Steuerung verbunden werden, die sicherstellt, dass der Plunger nicht an die axialen Endwand des Arbeitsraums stößt bzw. die Gewindespindel oder die Spindelmutter über ihre Endpositionen hinaus verschoben oder gedreht werden.

### Kurze Beschreibung der Zeichnungen

Figur 1 ist eine schematische Schnittansicht einer ersten Ausführungsform der automatisierten hochpräzisen Plungerpumpe gemäß der Erfindung; und
Figur 2 ist eine schematische Schnittansicht einer zweiten Ausführungsform der automatisierten hochpräzisen Plungerpumpe gemäß der Erfindung.

### Wege zur Ausführung der Erfindung

Im Folgenden werden bevorzugte Ausführungsformen der vorliegenden Erfindung unter Bezugnahme auf die Zeichnungen beschrieben. Zunächst wird zum besseren Verständnis des Einsatzbereichs der Erfindung die automatisierte Isolierung beliebiger einzelner oder seltener Zellen beschrieben, welche in drei Schritten abläuft: Zellerkennung; Zellsammlung und Zellabgabe.

Die Erfinder haben zu diesem Zweck ein System bereitgestellt, das auf einem inversen Mikroskop (Umkehrmikroskop), einer Zellerkennungseinheit, einer automatisierten Kapillarverstellung, einer automatisierten Pumpe und einem Verschiebetisch basiert. Die Sammlung und Abgabe werden mithilfe einer (im Folgenden detailliert beschriebenen) hochpräzisen Pumpe gesteuert, die es erlaubt, Verfahrensabläufe festzulegen, die Nanoliter-Volumen eines Pumpmediums für den Abtrennvorgang der Zelle einsetzen und so die Grundlage schaffen für eine am Verfahrensende durchzuführende molekulare Analyse des Zellmaterials in lediglich 1 Microliter Medium.

Die (nicht-haftenden) Zellen erfahren dabei keinerlei mechanische Belastungen: die Zellsammlung erfolgt lediglich aufgrund des die Zelle umgebenden Flüssligkeitsflusses. Unter optimalen Bedingungen können selbst teilweise anhaftende Zellen auf diese Weise gesammelt werden. Es besteht kein Kontakt zwischen den Zellen und der zur Sammlung eingesetzten Kapillare. Der Kapillardurchmesser kann wesentlich größer sein als jener der Zelle. Zum Beispiel können Zellen mit 6 µm Durchmesser effizient mit einer Kapillare von 40 µm Durchmesser gesammelt werden.

Die Abgabe der Zellen kann auf verschiedene Zielträger (deposits) erfolgen. Sogenannte "Grid deposits" sind dabei entweder raster-artige Punktablagen (wie zum Beispiel AmpliGrid^{®}) oder raster-artig angeordnete kleine Behälter (wie die IBIDI Probentaschen-Objektträger). Einzelpunktablagen können aus einem transparenten Deckel, einem PCR-Röhrchen oder einer Mikrofluid-Vorrichtung bestehen. Wie auch immer der Zielträger beschaffen ist, seine Größe sollte im Allgemeinen nicht die Größe von Standard-Objektträgern überschreiten, damit diese in die Mehrfachhalterung der Objektträger auf dem Verschiebetisch eingesetzt werden können.

Die Sammlung und Abgabe der Zellen kann auf verschiedene Arten erfolgen: von der manuellen bis zur voll automatisierten Betriebsart mit Zellerkennung. Jedoch ist es selbst bei der manuellen Betriebsart nicht notwendig, irgendeine Komponente des Systems (Mikroskop, Pumpe, Kapillare) von Hand zu bedienen: alle Betriebsvorgänge werden durch den Benutzer vom PC aus gestartet.

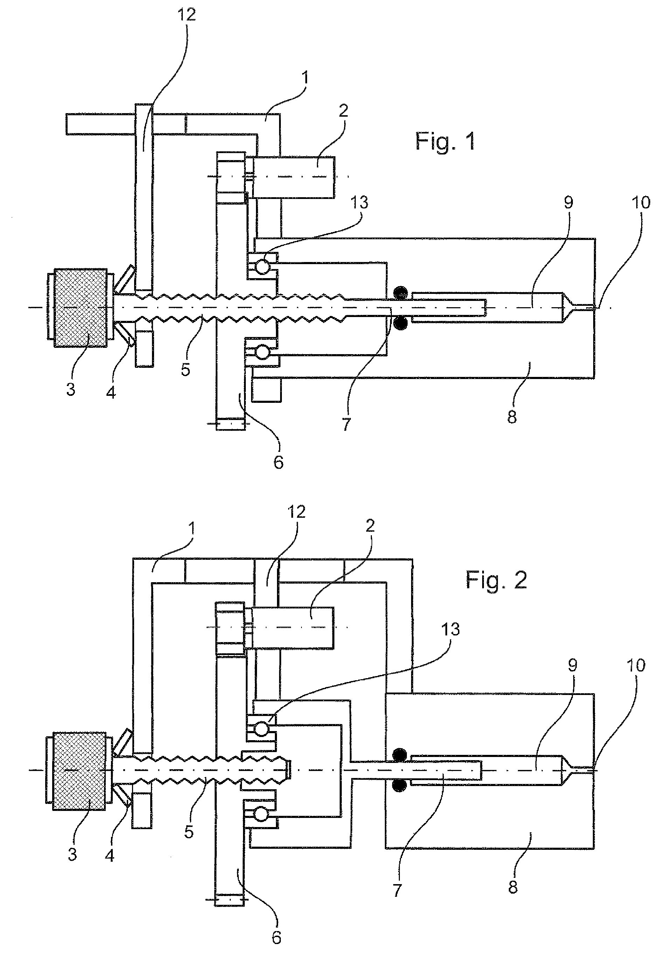

Anhand der Figur 1, die eine schematische Schnittansicht einer ersten Ausführungsform der erfindungsgemäßen Plungerpumpe zeigt, wird nun die für die Sammlung und Abgabe der Zellen wesentliche Funktionsweise der Plungerpumpe beschrieben. Die Plungerpumpe umfasst ein Gehäuse 1 und einen fest am Gehäuse befestigten Pumpkopf 8. Im Pumpkopf 8 befindet sich ein Arbeitsraum 9, in welchem sich im Betrieb das Pumpmedium, zum Beispiel ein Öl oder eine für das zu sammelnde biologische Material inerte Flüssigkeit. Es ist aber auch denkbar, Luft oder ein Gas als Pumpmedium einzusetzen. Der Arbeitsraum ist im vorliegenden Ausführungsbeispiel länglich und einer Spritze ähnlich ausgebildet und besitzt an einem Ende eine Ein- und Auslassöffnung, durch die Pumpmedium angesaugt oder ausgestoßen wird.

Am anderen Ende des länglichen Arbeitsraum 9 ist über eine Dichtung ein Plunger (Tauchkolben) 7 eingeführt, der sich im Arbeitsraum axial (d.h. entlang der Längsausdehnung des Arbeitsraums) bewegen kann und durch sein eigenes Volumen das entsprechende Volumen des im Arbeitsraum befindlichen Fluids verdrängt. In anderen Worten kann durch Hin- und Herbewegen des Plungers 7 das verfügbare Volumen des Arbeitraums verändert werden. An seinem dem Arbeitsraum abgewandten Ende ist der Plunger 7 mit einer Gewindespindel 5 verbunden, wobei diese Verbindung in Figur 1 starr gezeigt ist, jedoch auch über ein Lager frei drehend ausgebildet sein kann.

Die Gewindespindel 5, die in einer im Gehäuse axial beweglichen Halterung 12 gelagert ist, ist relativ zum Gehäuse ebenfalls verschiebbar angeordnet und greift über ihr Gewinde mit einer Spindelmutter 6 ein, welche unverschieblich, aber drehbar im Gehäuse 1 (oder im Pumpkopf 8) gelagert ist. Die Spindelmutter 6 steht ihrerseits mit der Welle eines Elektromotors 2 in Gewindeeingriff und wird von diesem zur Drehung angetrieben. Zudem ist am vom Plunger 7 entfernten Ende der Gewindespindel 5 ein Handrad 3 zum manuellen Antrieb der Pumpe vorgesehen.

Zwischen dem Handrad 3 und der Halterung 12 befindet sich eine Rutschkupplung 4, welche beispielsweise durch die Federkraft einer Wellenscheibe einer Rotation des Handrads 3 und damit der Gewindespindel 5 entgegenwirkt und im Normalbetrieb verhindert.

Im Betrieb wird also die Spindelmutter 6 durch den Elektromotor 2 in Drehung versetzt, und diese Drehung der unverschieblichen Spindelmutter 6 wird in eine Längsbewegung der nicht rotierenden (arretierten) Gewindespindel 5 umgewandelt, welche wiederum den Plunger 7 der Pumpe axial im Arbeitsraum verschiebt. Durch den direkten Antrieb der Gewindemutter 6 kann damit eine sehr genaue und kontrollierbare Verschiebung des Plungers 7 erhalten werden, die einer sehr genau definierten Volumenänderung im Nanoliter-Bereich entspricht. Mit dieser sehr genauen Volumenänderung lässt sich wiederum ein exakt definierter Fluss an Pumpmedium erzeugen, mithilfe dessen einzelne Zellen oder Zellbestandteile abgetrennt und gesammelt werden können.

Sollte es im Betrieb notwendig sein, den Fluidfluss bzw. das Pumpvolumen zu korrigieren oder das Pumpmedium nachzufüllen, so kann die Plungerpumpe über das Handrad 3 betätigt werden. Dabei wird zunächst der Motor 2 und die Drehung der Spindelmutter 6 gestoppt. Durch Drehung des Handrads 3 wird die arretierende Wirkung der Rutschkupplung 4 überwunden und die Gewindespindel 5 im Gehäuse gedreht. Da der Motor 2 ausgeschaltet ist und sich die Spindelmutter 6 nicht dreht (arretiert ist), schraubt sich die Gewindespindel 5 durch die Mutter 6 und bewegt den Plunger 7 in axialer Richtung im Arbeitsraum 9.

Eine zweite Ausführungsform der erfindungsgemäßen Plungerpumpe ist in Figur 2 dargestellt. Im Unterschied zur ersten Ausführungsform der Figur 1 ist hier die Gewindespindel 5 nicht direkt, d.h. translatorisch, an den Plunger 7 gekoppelt. Vielmehr ist der Plunger 7 über ein Wälzlager mit der Spindelmutter 6 verbunden, welche zusammen mit ihrem Antriebsmotor 2 axial im Gehäuse 1 beweglich angeordnet ist, also nicht am Gehäuse 1 fixiert ist, und sich somit zusammen mit dem Plunger axial verlagert. Die Gewindespindel 5 hingegen ist translatorisch fest am Gehäuse 1 angebracht und über eine Reibkupplung rotatorisch fixiert. Bei Betrieb des Motors 2 dreht sich die Spindelmutter 6 und verfährt also axial auf der von der Reibkupplung rotatorisch fixierten Gewindespindel 5. Dabei bewegt die Spindelmutter 6 auch den Plunger 7 in axialer Richtung im Arbeitsraum 9.

Sollte bei den Plungerpumpen der beiden in Figur 1 bzw. Figur 2 beschriebenen Ausführungsformen ein übermäßiges Kraftmoment auf die Gewindespindel 5 einwirken, so rutscht die Rutschkupplung 4 durch und erlaubt die Drehung der Gewindespindel 5, so dass die traslatorische Bewegung der Spindelmutter 6 gestoppt wird. Auf diese Weise ist sichergestellt, dass die empfindliche Mechanik der Plungerpumpe nicht aufgrund von Bedienungsfehlern oder anderen Umständen beschädigt wird.

## Patentansprüche

1. Automatisierte hochpräzise Plungerpumpe für Volumen unter einem Mikroliter, umfassend
ein Gehäuse (1) und einen Pumpkopf (8), der einen Arbeitsraum (9) mit einer gemeinsamen oder separaten Ein- und Auslassöffnung (10) definiert, wobei das Volumen des Arbeitsraums (9) durch einen in ihn einbringbaren axial beweglichen Plunger (7) veränderbar ist
**dadurch gekennzeichnet, dass**
der Plunger (7) durch eine Gewindespindel (5) oder eine auf der Gewindespindel (5) in Gewindeeingriff befindliche Spindelmutter (6) axial beweglich ist, und
die Gewindespindel (5) und/oder die Spindelmutter (6) rotatorisch antreibbar und die jeweils andere rotatorisch arretierbar ausgelegt ist.

2. Plungerpumpe nach Anspruch 1, wobei die Plungerpumpe einen Motor (2) umfasst, der mit der Spindelmutter (6) und/oder der Gewindespindel (5) in Eingriff steht und über sie mit der rotatorisch arretierten Gewindespindel (5) bzw. der rotatorisch arretierten Spindelmutter (6) zum Antrieb des Plungers (7) in Wirkverbindung steht.

3. Plungerpumpe nach Anspruch 1 oder 2, wobei die Gewindespindel (5) und/oder die Spindelmutter (6) mit einem vorgeschalteten Getriebe versehen sind.

4. Plungerpumpe nach einem der vorangehenden Ansprüche, wobei die arretierbare Gewindespindel, die arretierbare Spindelmutter oder der Motor (2) mit einer Kupplung (4) versehen ist.

5. Plungerpumpe nach Anspruch 4, wobei die Kupplung (4) eine Rutschkupplung ist.

6. Plungerpumpe nach Anspruch 4 oder 5, wobei die Kupplung (4) manuell oder automatisiert ist.

7. Plungerpumpe nach einem der vorangehenden Ansprüche, wobei die Spindelmutter (5) gegenüber dem Gehäuse (1) oder gegenüber dem Plunger (7) über ein Wälzlager (13) gelagert ist, welches spielfrei vorgespannt ist.

8. Plungerpumpe nach einem der vorangehenden Ansprüche, wobei der Plunger (7) mit der Gewindespindel (5) mitdrehend, insbesondere drehstarr verbunden ist.

9. Plungerpumpe nach einem der vorangehenden Ansprüche, wobei der Pumpkopf mit in den Arbeitsraum führenden, bevorzugt richtungsorientierten Ventilen versehen ist.

10. Plungerpumpe nach einem der vorangehenden Ansprüche, wobei die Endlagen des Plungers (7) im Arbeitsraum (9) angebende Endschalter im Pumpenkopf (8) vorgesehen sind.
